# EUROPEAN PATENT SPECIFICATION

(11) **EP 0 662 911 B1**
(45) Date of publication and mention of the grant of the patent: **26.03.1997**
(21) Application number: 93921019.1
(22) Date of filing: 28.09.1993
(51) Int. Cl.: B65D 1/04, B65D 35/22

(54) **DISPENSING PACKAGE WITH CLOSURE SYSTEM**
SPENDERVERPACKUNG MIT VERSCHLUSSSYSTEM
BOITE DE DISTRIBUTION PRESENTANT UN SYSTEME DE FERMETURE

(30) Priority: 30.09.1992 US 954847; 30.09.1992 US 954848
(43) Date of publication of application: 19.07.1995
(73) Proprietor: UNILEVER PLC, London EC4P 4BQ (GB); UNILEVER N.V., 3013 AL Rotterdam (NL)
(72) Inventor: GENTILE, James, Louis, Orange, CT 06477 (US); WILLIAMS, David, Robert, Monroe, CT 06468 (US); ZIEMKIEWICZ, Alexander, George, Shelton, CT 06484 (US)
(74) Representative: Gordon, Naoise Padhraic Edward
(86) International application number: GB9302023
(87) International publication number: WO9407748

(56) References cited:
- WO-A-92/04007
- GB-A- 2 248 820
- US-A- 3 705 661
- US-A- 4 148 417
- US-A- 4 884 703

## Description

This invention relates to a package, such as a container and closure, from which at least two substances can be dispensed substantially simultaneously. In a preferred embodiment of the invention, the package may be used to contain an effervescible mouthwash composition which generates fresh effervescence at the time of use.

Dispensing packages, containers and the like have been proposed for dispensing two or more different substances or materials substantially simultaneously. For example, in U.S. 4,964,539 there is disclosed a multiple chamber, dispensing container and closure in the form of a multi-compartment tube and cap. U.S. 4,687,663 discloses an article for storage and delivery of a two-part dental preparation from which controlled quantities of the semi-solid preparation may be dispensed.

U.S. 3,729,553 discloses an effervescent composition packaged in a container having two compartments for storage of ingredients of the composition, and from which the ingredients are said to be dispensed simultaneously and proportionately. In U.S. 3,729,553 the components of the composition are stored separately to prevent premature mixing of the components and gas release, with the components subsequently being simultaneously and proportionately dispensable. However, the packaged effervescent composition disclosed is silent on features which would provide guidance to a user on how to use the pack to avoid unnecessary pre-mixing.

Multi-compartment packaging is generally employed when it is necessary to keep the components of a composition separated until used, to prevent premature reaction.

A problem with multi-compartment packaging intended for dispensing and storing liquid components is that following use there is a tendency for one or a mixture of the components to drain back into the compartments and spoil the contents. A further problem is that the user of the package is given insufficient visual guidance on how to dispense the components from the package, which can lead to unwanted premature mixing of the materials during pouring. Such a problem can be particularly acute when the multicompartment package is used to store a two component system which generates a gas such as oxygen on the mixing of the components, such as a two component mouthwash composition. This is because unwanted premixing of the components can occur, thereby causing gas evolution not to occur in the place where it is most wanted, i.e. in the users mouth.

Thus, it would be desirable to provide a package with improved pouring and draining capabilities. Further, it would be beneficial if the package could be provided with an easily operated closure system that could be mounted on or otherwise integrated with the container in which the components are stored and which can provide visual guidance on how to dispense the components from the package. In one embodiment this package can be used to store a packaged effervescible mouthwash composition which has improved dispensing capabilities, and is less prone to premature reaction.

Thus according to one aspect of the invention, there is provided a package for dispensing at least two liquid components simultaneously, see claim 1.

According to a further aspect of the invention, there is also provided a closure system for the same purpose, see claim 6.

In certain preferred embodiments, the cover for securement to the crown portion is flat.

In a preferred embodiment, the container as described above may be used to provide a packaged effervescible mouthwash composition comprising a first liquid component containing a functional ingredient and a second liquid component containing a functional ingredient, the functional ingredients in the liquid components on mixing being capable of generating oxygen.

In yet a further preferred embodiment, in the packaged effervescible mouthwash composition the functional ingredient in the first liquid component may comprise a peroxide compound. Most preferably, the peroxide compound in hydrogen peroxide. In this embodiment the functional ingredient in the second liquid component may comprise a bicarbonate salt, preferably an alkali or alkali earth metal bicarbonate, and may most preferably be sodium bicarbonate.

The pouring spouts are preferably inclined, so as to effect a pouring tip at the lower end of the spout. Preferably each pouring spout is provided with a vent opening located towards the upper edge of the crown, which allows smaller through opening sizes to be used than would be possible in their absence. Without the vent openings the through openings must be large enough to allow air to enter the container, in order to prevent "glugging" and uneven pouring. With vent openings the through openings can be made smaller, and therefore positioned closer together on the crown. This gives the advantage that the package can be used for dispensing more than one liquid from one package into a small container, such as a mouthwash cup, with controlled pouring.

The inclined crown of the closure and positioning of the pouring spouts towards the lower edge of the crown visually directs the user of the package to dispense liquid over the lower edge of crown only. This visual direction is enhanced by pouring spouts which are inclined to form a pouring tip. Pouring over the lower edge of the crown prevents premature mixing of the liquid streams. The spouts also aid the drain back of the components only into the compartment from which they were poured, and helps prevent drain back of a mixture of the components into a compartment.

Other advantages and features of the present invention will become readily apparent from the following detailed description of the invention, from the claims, and from the accompanying drawings.

In the accompanying drawings which will be used to demonstrate the invention by way of example only, like numerals are employed to designate like parts.

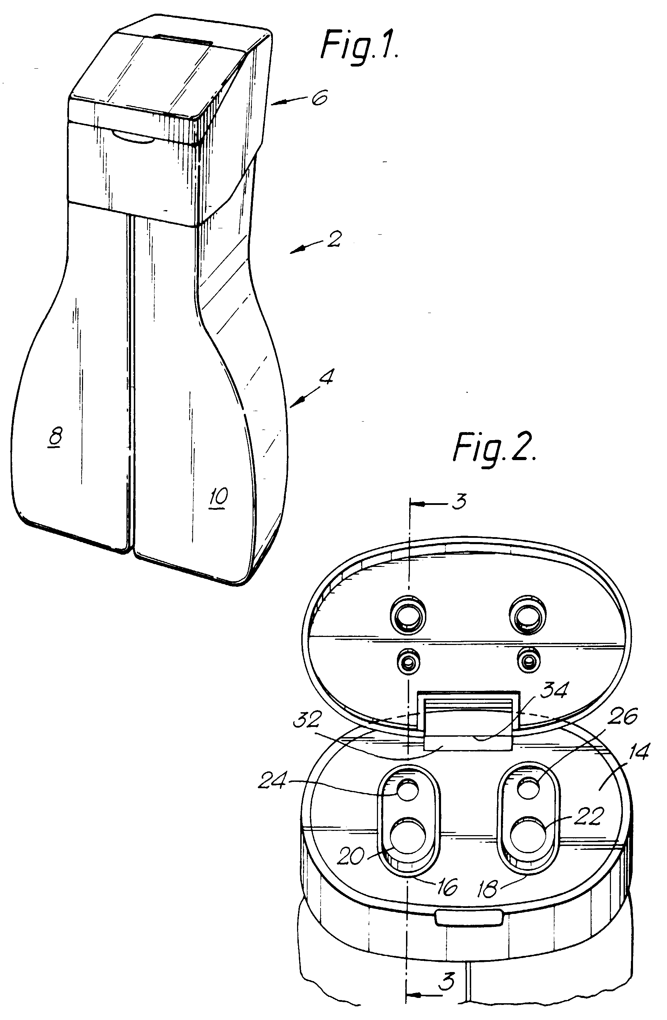

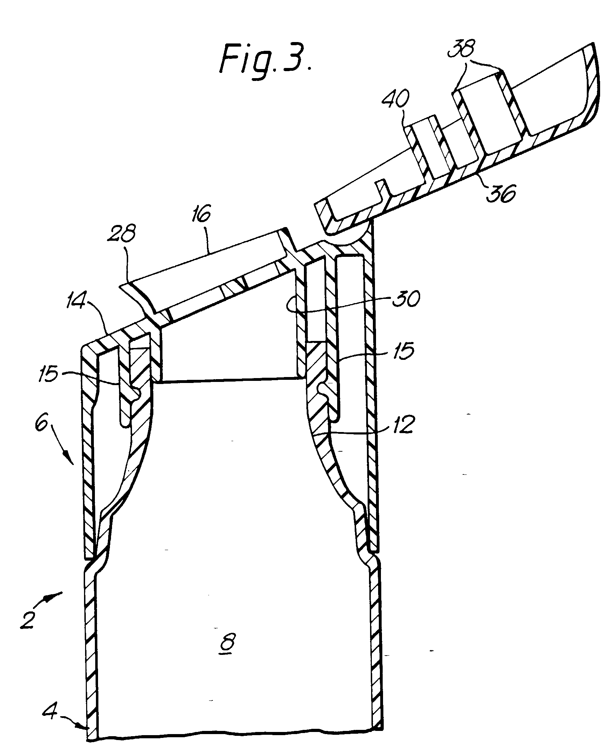

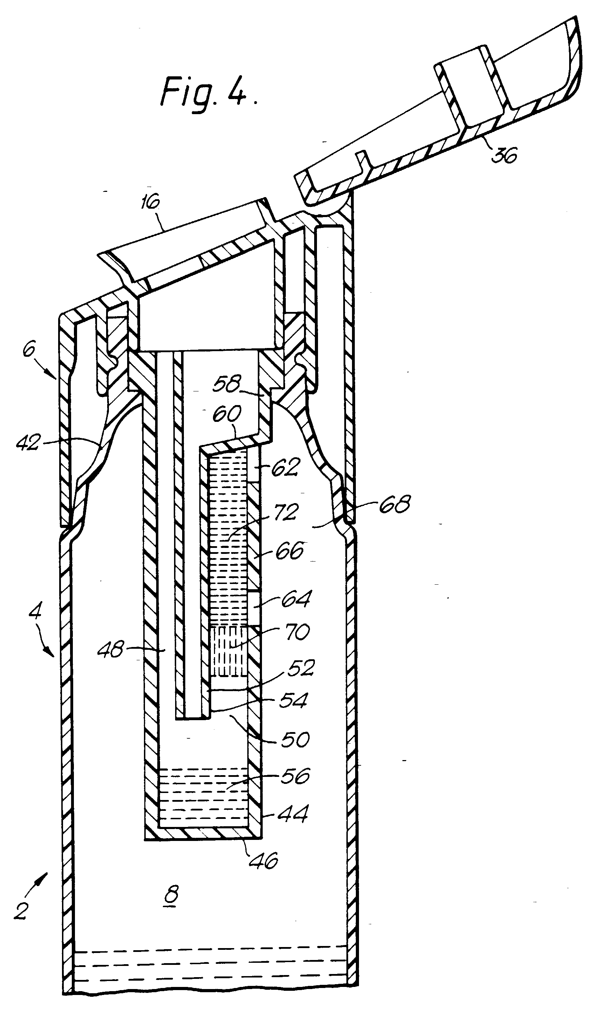

Figure 1 is a perspective view of the package of the present invention shown with the cover in the closed position. Figure 2 is a top plan view of an open closure which may be mounted on a container, such as is shown in Figure 1. Figure 3 is a fragmentary, cross-sectional view of the closure of Figure 2 mounted on a container taken generally along the plane 3-3 of Figure 2. Figure 4 is a fragmentary, cross-sectional view of the closure of Figure 2 mounted on a container with a dosing device.

For ease of description, the article of this invention is described in an upright position, and terms such as upper, lower, inclined etc. are used with reference to this position. It will be understood, however, that the article of this invention may be manufactured, stored, transported and sold in an orientation other than the position described.

A first embodiment of a package in accordance with the teachings of the present invention is designated generally in Figure 1 by the reference numeral 2. The package 2 comprises a container 4 and a closure 6. The container 4 comprises separate storage compartments 8, 10. Each compartment 8, 10 terminates upwardly with an outlet end 12 as can be more clearly seen in Figure 3. The two-compartment container 4 can either be formed of two entirely separate compartments 8 and 10 which are held together by the closure system 6 as shown in Figure 1, or can be formed by a dividing wall in the container.

The bottom of the container 4 may have any suitable configuration. Conveniently it is at least partially flat, to facilitate standing on a horizontal surface.

Figure 2 and Figure 3 show the closure system 6 comprising an inclined crown portion 14 having a peripheral skirt portion 15 depending downwardly from an outer edge of the crown, to engage the outer surface of the container 4 in a fluid tight manner. To this end, the outer surface of the container 4 may be provided with an annular concave groove, and the inner surface of the skirt 15 may be provided with an inwardly projecting annular protuberance, bead or ring for engagement in the groove. This provides a conventional groove and bead snap-fit engagement, which can be substituted with known equivalent engagements or seals.

Two pouring spouts 16, 18 extend upwardly from the upper surface of the crown and are located toward the lower edge of the crown 14. The pouring spouts 16, 18 are each provided with a through opening 20, 22 which extends from the spout 16, 18, through the crown 14 and into a compartment 8, 10. Each spout is further provided with a vent opening 24, 26 which extends from the spout 16, 18, through the crown 14 and into a compartment 8, 10. Each spout 16, 18 is inclined to effect a pouring tip 28 shown in Figure 3 for spout 16.

In one preferred aspect of the invention, spouts 16, 18 are substantially oval in shape, and may have their longitudinal axes parallel and extending between the lower and upper edges of the crown.

The inclined crown portion 14 has, in addition to the peripheral skirt portion 15, an inner skirt portion 30, as shown in Figure 3, to engage the inner surface of the container 4 in a fluid tight manner.

The inclined crown portion 14 may have, towards its upper edge, a minor portion 32 of reversed incline to that of the major portion of the crown 14, which portion 32 bears a hinge means 34 for a flat cover 36, as shown in Figure 3. Figures 2 and 3 show an inclined crown portion with a rotatable hinge 34 for a flat cover 36.

In the preferred embodiment illustrated in Figures 1-3 the cover 36 is connected to the crown 14 via a snap-action hinge 34 that may be of a conventional molded plastic design. When the cover 36 is closed it is maintained in this position by the hinge. When the cover 36 is open it is maintained in this position, to aid dispensing of the liquids with one hand. It will readily be appreciated that the cover 36 may be hingedly attached to the crown by many alternative means.

Flat cover 36 is provided with four depending plugs receivable in the through and vent openings 20, 22, 24, 26 of the spouts 16, 18. The plugs 38, 40 for through opening 20 and vent opening 24 of spout 16 are shown in Figure 3. When the flat cover 36 is in the closed position as shown in Figure 1, the plugs close the through and vent openings and seal the closure system and package. When the flat cover 36 is in the open position, liquids in compartments 8 and 10 are simultaneously dispensed by tilting the container 4 to a substantially horizontal position so that the liquids flow from the spouts 16, 18 over the lower edge of the crown 14. Each of the liquids flow by gravity from the container in a substantially uniform flow.

Preferably, the container 4 and closure 6 are each made of plastics materials. Conveniently container 4 is made by blow molding, whilst closure 6 is injection molded. Conveniently container 4 is opaque.

In an alternative embodiment, flat cover 36 may be replaced by, or have in addition to the flat cover 36 attached to the crown 14 a removable cup, which can also serve as a measuring cup, and is retained on crown 14 by retaining lugs.

An additional embodiment of the present invention is illustrated in Figure 4 and is substantially similar to the first embodiment illustrated in Figures 1-3, except that each container 8, 10 is additionally provided with a dosing device which aids the pouring of measured equal quantities of the liquids out of the package. Figure 4 shows a dosing device installed in compartment 8 of the container 4 in the neck 42 thereof. The device projects into the interior of the container 4 while forming a seal with the container neck 42 by reason of the outer end of the dosing device fitting closely within the container neck 42.

The dosing device has at its inner end 44 a bottom 46. Two passages 48, 50 which are separated from one another by a partition wall 52, extend in the axial direction in the interior of the dosing device. The lower end 54 of the partition wall 52 is spaced from the bottom 46 to form a receptacle 56 which brings the two passages 48, 50 into communication with one another within the closed inner end 44 of the dosing device. The volume of the receptacle 56 is at least as large as the volume of liquid to be dispensed. The passage 48 acts as a through opening which is open at the spout 16 of the closure device 6.

The other passage 50 is shut off from the outer end 58 of the dispensing device by transverse wall 60. Two apertures 62, 64 which are spaced apart in the axial direction of the passage 50, are provided in the outer wall 66 of the passage 50. The passage 50 communicates with the interior of the container through the two apertures 62 and 64, the outer wall 66 of the passage 50 being spaced from the neck of the container by a gap 68.

Between the transverse wall 60 and the aperture 64 the passage 50 provides a measuring chamber, the volume of which is equal to the volume of liquid dispensed. The passage 50 also provides a collecting chamber between the aperture 64 and the end 54 of the partition wall, the volume of which is at least as great as half the maximum free liquid which occurs.

The mode of operation of the dosing device is as follows. When the container 4 is tilted, liquid flows from its interior through the gap 68 and through the aperture 62 into the passage 50, and at the same time the displaced air escapes from passage 50 through the aperture 64 into the interior of the bottle until the metering chamber 72 is filled with liquid to be dispensed as indicated by horizontal hatching in the drawing. When aperture 64 is so closed, no more air can enter the container. Only free liquid now flows into the collecting chamber 70, as shown by vertical hatching.

As a result of the flow of free liquid the air in the interior of the bottle is at reduced pressure. If the container is returned to the vertical, the air at reduced pressure draws liquid out of the passage 50 through the aperture 64 into the interior of the container until the pressure is equalized. In consequence the measured quantity of liquid flows out of the metering chamber 72 into the receptacle 56. During the next tilting of the container 4 this quantity of liquid is poured out of the dosing device through the passage 48 and spout 16. In the course of this, the next metering operation takes place in the passage 50. While Figure 4 shows the dosing device installed in one compartment of the container, each compartment of the container will normally contain a dosing device.

It will be readily observed from the foregoing detailed description and from the illustrated embodiments thereof that numerous variations and modifications may be effected without departing from the true spirit and scope of the principles of this invention.

As described above, it is a preferred embodiment of the invention that the package as described above be used to provide a packaged effervescible mouthwash composition, comprising two functional ingredients in different liquid components which when mixed are capable of generating oxygen. The functional ingredient of the first liquid component of the composition preferably comprises a peroxide compound, most preferably hydrogen peroxide. The functional ingredient of the second liquid component of the composition preferably comprises a bicarbonate salt, more preferably on alkali or alkali earth metal bicarbonate, most preferably sodium bicarbonate.

Preferably the first liquid component comprises from 0.5 to 5% by weight of a peroxide, more preferably 1 to 5%. The second liquid component preferably comprises from 1 to 5% by weight of a bicarbonate, more preferably 2 to 5%.

Other oral hygiene medicaments suitable for use in a mouthwash product may be used in packaged mouthwash products according to the present invention, such as anti-caries agents, anti-calculus agents, anti-plaque agents, anti-microbial agents or the like.

Suitable anti-caries agents include fluoride ion sources, such as alkali metal fluorides, alkali metal monofluorophosphates, stannous fluoride, most preferably sodium fluoride. Fluoride ion sources may be used in an amount sufficient to provide from about 25 ppm to about 1000 ppm fluoride, based on the total weight of the mouthwash.

Suitable anti-calculus agents include the linear molecular dehydrated polyphosphate salts, including alkali metal tripolyphosphates and pyrophosphates. Preferably, the polyphosphate anti-calculus agent may be used in an amount of up to about 5%, preferably from about 0.5 to about 2%, by weight, based on the total weight of the mouthwash. The packaged effervescible mouthwash composition may include any suitable conventional ingredients. The ingredients commonly employed in mouthwash compositions include, for example, flavouring agents, anti-foam agents, alcohols, antimicrobial agents, sweetening agents, surface active agents, deodorizing agents, colouring agents, bactericidal agents, astringent agents and the like. Any number of the foregoing ingredients, as well as other conventional ingredients, may be present in the compositions of this invention.

The quantity of peroxide and bicarbonate employed in the liquid components will depend upon the desired quantity of oxygen to be made available from the two-liquid system, but preferably 4% by weight will be present in each compartment.

Generally, the mouthwash of the invention will comprise from about 45 to about 95%, by weight of water, based on the total weight of the mouthwash.

Conventional manufacturing techniques may be used to prepare the package and mouthwash of the invention.

The present invention is illustrated in terms of its preferred embodiments in the accompanying Examples. All parts and percentages referred to in this specification are by weight, based upon the total weight of the mouthwash, unless otherwise specified.

### EXAMPLE 1

A hydrogen peroxide containing liquid and a sodium bicarbonate containing liquid were prepared by admixing the following ingredients:

| **Ingredients** | **H**_{**2**}**O**_{**2**} **Liquid** | **NaHCO**_{**3**} |
|---|---|---|
| Deionized water | balance | balance |
| Ethanol | | 24 |
| Humectant (Polyol 2) | | 7 |
| Solubilizer (polysorbate) | | 0.4 |
| Flavour | | 0.4 |
| Sodium bicarbonate | | 2 |
| Hydrogen peroxide(35% sol.) | 4.285 | |
| Dye | 0.0025 | |
| Saccharin | | 0.065 |
| Sodium lauryl sulphate | | 0.6 |
| Phosphoric acid | 0.04 | |

Equal amounts of the liquids were added to compartments 8 and 10 of a package of the type shown in Figures 1 to 3.

The liquids after being dispensed from the package combined to form an effervescent mouthwash.

## Claims

1. A package (2) for dispensing at least two liquid components simultaneously, the package (2) comprising:
a container (4) for the components, the container having at least two discrete compartments (8,10) each with an upper outlet end (12) and
a closure system (6) for closing the container (4) over the outlet end (12) of the compartments (8,10) characterised in that the closure (6) comprises:
an inclined crown portion (14) having a peripheral skirt portion (15) depending downwardly from an outer edge of the crown (14), the skirt (15) portion being of sufficient size to engage a surface of the container (4) in a fluid tight manner;
at least two pouring spouts (16,18) extending upwardly from the upper surface of the inclined crown portion (14), toward the lower edge thereof; the spouts (16,18) being substantially oval in shape and having their longitudinal axes parallel and extending between the lower and upper edges of the inclined crown portion (14) in the direction of inclination thereof;
each pouring spout (16,17) being provided with a through opening (20,22) which extends from the upper end of the spout (16,18), through the crown (14) and into a compartment (8,10) and
a cover (36) for securement to the crown portion (14) being hingedly attached to the crown toward an upper edge thereof, the cover (36) being provided with at least two depending plugs (38) receivable in corresponding through openings (20,22) of the crown (14) so as to close the container (4).

2. A package according to claim 1 wherein the pouring spouts (16,18) are inclined to effect a pouring tip (28) at the lower end of the spout (16,18).

3. A package according to claim 1 or claim 2 wherein each pouring spout (16,18) is provided with a vent opening (24,26) which extends from the upper end of the spout (16,18), through the crown (14) and into a compartment (8,10).

4. A package according to any of the preceding claims, wherein each compartment (8,10) is provided with a dosing device.

5. A package according to any of the preceding claims, wherein the container (4) is divided into at least two separate compartments (8,10) by an internal dividing wall.

6. A closure system (6) for closing a container (4) for liquids having at least two discrete compartments (8,10) characterised in that the closure (6) comprises:
an inclined crown portion (14) having a peripheral skirt portion (15) depending downwardly from an outer edge of the crown, the skirt portion (15) being of sufficient size to engage a surface of the container (4) in a fluid tight manner;
at least two pouring spouts (16,18) extending upwardly from the upper surface of the inclined crown portion (14), toward the lower edge thereof; the spouts (16,18) being substantially oval in shape and having their longitudinal axes parallel and extending between the lower and upper edges of the inclined crown portion (14) in the direction of inclination thereof.
each pouring spout (16,18) being provided with a through opening (20,22) which extends from the upper end of the spout (16,18), through the crown (14); and
a flat cover (36) for the crown portion (14) being hingedly attached to the crown (14) toward an upper edge thereof, the cover (36) being provided with at least two depending plugs (38) receivable in corresponding through openings (20,22) of the crown (14) so as to close the container (4).

7. A package according to any of claims 1-5, further comprising an effervescible mouthwash composition therein which composition comprises a first liquid component containing a functional ingredient and a second liquid component containing a functional ingredient, the liquid components on mixing being capable of generating oxygen.

8. A package according to claim 7, wherein the functional ingredient in the first liquid component is a peroxide and the functional ingredient in the second liquid component is bicarbonate.

9. A package according to claim 8, wherein the first liquid component comprises from 0.5-5% by weight of a peroxide.

10. A package according to claim 8, wherein the second liquid component comprises from 1 to 5% by weight of bicarbonate.

## Patentansprüche

1. Verpackung (2) zur gleichzeitigen Ausgabe von mindestens zwei flüssigen Komponenten, wobei die Verpackung (2) umfaßt:
einen Behälter (4) für die Komponenten, wobei der Behälter mindestens zwei voneinander getrennte Kammern (8,10) jeweils mit einem oberen Auslaßende (12) aufweist, und
ein Verschlußsystem (6) zum Verschließen des Behälters (4) über dem Auslaßende (12) der Kammern (8,10), dadurch gekennzeichnet, daß der Verschluß (6) umfaßt:
ein abgeschrägtes Aufsatzteil (14) mit einem peripheren Einfassungsteil (15), das abwärts von der Außenkante von Aufsatz (14) herabhängt, wobei Einfassungsteil (15) von ausreichender Größe ist, um in eine Fläche des Behälters (4) flüssigkeitsdicht einzufassen;
mindestens zwei Gießtüllen (16,18), die sich aufwärts von der oberen Fläche des abgeschrägten Aufsatzteils (14) zu der unteren Kante davon erstrecken; wobei die Tüllen (16,18) im wesentlichen ovale Form aufweisen und parallel ausgerichtete Längsachsen aufweisen, und sich zwischen der unteren und oberen Kante des abgeschrägten Aufsatzteils (14) in Richtung von dessen Abschrägung erstrecken;
wobei jede Gießtülle (16,17) mit einer Durchgangsöffnung (20,22) ausgestattet ist, die sich vom oberen Ende der Tülle (16,18) durch den Aufsatz (14) und in eine Kammer (8,10) erstreckt, und
einen Deckel (36) zur Befestigung auf dem Aufsatzteil (14), der an dem Aufsatz gegen eine obere Kante davon aufklappbar befestigt ist, wobei der Deckel (36) mit mindestens zwei herabhängenden Stöpseln (38) versehen ist, die von den entsprechenden Durchgangsöffnungen (20,22) des Aufsatzes (14) aufgenommen werden können, so daß der Behälter (4) geschlossen wird.

2. Verpackung nach Anspruch 1, wobei die Gießtüllen (16,18) abgeschrägt sind, um eine Gießspitze (28) am unteren Ende der Tülle (16,18) auszubilden.

3. Verpackung nach Anspruch 1 oder Anspruch 2, wobei jede Gießtülle (16,18) mit einer Entlüftungsöffnung (24,26) ausgestattet ist, die sich vom oberen Ende der Tülle (16,18) durch den Aufsatz (14) und in eine Kammer (8,10) erstreckt.

4. Verpackung nach einem der vorangehenden Ansprüche, wobei jede Kammer (8,10) mit einer Dosiervorrichtung ausgestattet ist.

5. Verpackung nach einem der vorangehenden Ansprüche, wobei der Behälter (4) durch eine innere Trennwand in mindestens zwei getrennte Kammern (8,10) aufgeteilt ist.

6. Verschlußsystem (6) zum Verschließen eines Behälters (4) für Flüssigkeiten mit mindestens zwei getrennten Kammern (8,10), dadurch gekennzeichnet, daß der Verschluß (6) umfaßt:
ein abgeschrägtes Aufsatzteil (14) mit einem peripheren Einfassungsteil (15), das abwärts von der Außenkante des Aufsatzes herabhängt, wobei Einfassungsteil (15) von ausreichender Größe ist, um in eine Fläche des Behälters (4) flüssigkeitsdicht einzufassen;
mindestens zwei Gießtüllen (16,18), die sich aufwärts von der oberen Fläche des abgeschrägten Aufsatzteils (14) zu der unteren Kante davon erstrecken; wobei die Tüllen (16,18) im wesentlichen ovale Form aufweisen und parallel ausgerichtete Längsachsen aufweisen, und sich zwischen der unteren und oberen Kante des abgeschrägten Aufsatzteils (14) in Richtung von dessen Abschrägung erstrecken;
wobei jede Gießtülle (16,18) mit einer Durchgangsöffnung (20,22) ausgestattet ist, die sich vom oberen Ende der Tülle (16,18) durch den Aufsatz (14) erstreckt, und
einen flachen Deckel (36) für das Aufsatzteil (14), das an dem Aufsatz (14) gegen eine obere Kante davon aufklappbar befestigt ist, wobei der Deckel (36) mit mindestens zwei herabhängenden Stöpseln (38) versehen ist, die von den entsprechenden Durchgangsöffnungen (20,22) des Aufsatzes (14) aufgenommen werden können, so daß der Behälter (4) geschlossen wird.

7. Verpackung nach einem der Ansprüche 1-5, die außerdem ein sprudelfähiges Mundwassermittel darin umfaßt, wobei das Mittel mindestens eine erste flüssige Komponente, die einen funktionellen Bestandteil enthält, und eine zweite flüssige Komponente, die einen funktionellen Bestandteil enthält, umfaßt, wobei die flüssigen Komponenten nach Vermischen Sauerstoff erzeugen können.

8. Verpackung nach Anspruch 7, wobei der funktionelle Bestandteil in der ersten flüssigen Komponente ein Peroxid ist und der funktionelle Bestandteil in der zweiten flüssigen Komponente Bicarbonat ist.

9. Verpackung nach Anspruch 8, wobei die erste flüssige Komponente 0,5-5 Gewichtsprozent eines Peroxids umfaßt.

10. Verpackung nach Anspruch 8, wobei die zweite flüssige Komponente 1 bis 5 Gewichtsprozent Bicarbonat umfaßt.

## Revendications

1. Ensemble conditionné (2) destiné à distribuer simultanément au moins deux composants liquides, cet ensemble conditionné (2) comprenant:
un récipient (4) destiné aux composants, ce récipient possédant au moins deux compartiments distincts (8, 10) ayant chacun une extrémité supérieure (12) formant orifice de sortie et
un système (6) de fermeture destiné à fermer le récipient (4) au-dessus des extrémités (12) formant orifices de sortie des compartiments (8, 10)
caractérisé en ce que le dispositif de fermeture (6) comprend:
une partie inclinée (14) formant capsule qui possède une partie périphérique (15) formant jupe suspendue vers le bas à partir d'un bord externe de la capsule (14), la partie formant jupe (15) ayant une taille suffisante pour venir en contact avec une surface du récipient (4) d'une manière étanche aux fluides;
au moins deux goulottes verseuses (16, 18) s'étendant vers le haut à partir de la face supérieure de la partie inclinée (14) formant capsule, tout près du bord inférieur de cette dernière; ces goulottes (16, 18) étant de forme sensiblement ovale et ayant leurs axes longitudinaux parallèles et s'étendant entre les bords inférieur et supérieur de la partie inclinée (14) formant capsule dans la direction de l'inclinaison de cette dernière;
chaque goulotte verseuse (16, 18) étant pourvue d'une ouverture traversante (20, 22) qui s'étend à partir de l'extrémité supérieure de la goulotte (16, 18), à travers la capsule (14) et jusque dans un compartiment (8, 10) et
un couvercle (36), destiné à être assujetti sur la partie (14) formant capsule, étant fixé à la capsule avec une charnière située tout près d'un bord supérieur de cette capsule, ce couvercle (36) étant pourvu d'au moins deux bouchons suspendus (38) susceptibles de se loger dans les ouvertures traversantes (20, 22) de la capsule (14) correspondantes de manière à fermer le récipient (4).

2. Ensemble conditionné selon la revendication 1 dans lequel les goulottes verseuses (16, 18) sont inclinées de façon à réaliser un embout verseur (28) au droit de l'extrémité inférieure de la goulotte (16, 18).

3. Ensemble conditionné selon la revendication 1 ou la revendication 2 dans lequel chaque goulotte verseuse (16, 18) est pourvue d'une ouverture formant évent (24, 26) qui s'étend à partir de l'extrémité supérieure de la goulotte (16, 18), à travers la capsule (14) et jusque dans un compartiment (8, 10).

4. Ensemble conditionné selon l'une quelconque des revendications précédentes, dans lequel chaque compartiment (8, 10) est muni d'un dispositif de dosage.

5. Ensemble conditionné selon l'une quelconque des revendications précédentes, dans lequel le récipient (4) est divisé en au moins deux compartiments indépendants (8, 10) par une paroi intérieure de séparation.

6. Système (6) de fermeture destiné à fermer un récipient (4) pour liquides qui possède au moins deux compartiments distincts (8, 10) caractérisé en ce que le dispositif de fermeture (6) comprend:
une partie inclinée (14) formant capsule qui possède une partie périphérique (15) formant jupe suspendue vers le bas à partir d'un bord externe de la capsule, la partie (15) formant jupe ayant une taille suffisante pour venir en contact avec une surface du récipient (4) d'une manière étanche aux fluides;
au moins deux goulottes verseuses (16, 18) s'étendant vers le haut à partir de la face supérieure de la partie inclinée (14) formant capsule, tout près du bord inférieur de cette dernière; ces goulottes (16, 18) étant de forme sensiblement ovale et ayant leurs axes longitudinaux parallèles et s'étendant entre les bords inférieur et supérieur de la partie inclinée (14) formant capsule dans la direction de l'inclinaison de cette dernière;
chaque goulotte verseuse (16, 18) étant pourvue d'une ouverture traversante (20, 22) qui s'étend à partir de l'extrémité supérieure de la goulotte (16, 18), à travers la capsule (14); et
un couvercle plat (36), destiné à la partie (14) formant capsule, étant fixé à la capsule (14) avec une charnière située tout près d'un bord supérieur de cette capsule, ce couvercle (36) étant pourvu d'au moins deux bouchons suspendus (38) susceptibles de se loger dans les ouvertures traversantes (20, 22) de la capsule (14) correspondantes de manière à fermer le récipient (4).

7. Ensemble conditionné selon l'une quelconque des revendications 1 - 5, comportant en outre une composition pour bains de bouche susceptible de devenir effervescente qui s'y trouve, laquelle composition comprend un premier composant liquide contenant un ingrédient fonctionnel et un second composant liquide contenant un ingrédient fonctionnel, ces composants liquides étant aptes lors d'un mélange à générer de l'oxygène.

8. Ensemble conditionné selon la revendication 7, dans lequel l'ingrédient fonctionnel contenu dans le premier composant liquide est un péroxyde et l'ingrédient fonctionnel contenu dans le second composant liquide est du bicarbonate.

9. Ensemble conditionné selon la revendication 8, dans lequel le premier composant liquide contient de 0,5 à 5% en poids de péroxyde.

10. Ensemble conditionné selon la revendication 8, dans lequel le second composant liquide contient de 1 à 5% en poids de bicarbonate.
